# EUROPEAN PATENT APPLICATION

(11) **EP 1 790 295 A1**
(43) Date of publication of application: **30.05.2007**
(21) Application number: 05257251.8
(22) Date of filing: 25.11.2005
(51) Int. Cl.: A61B 17/11, A61M 29/00, A61B 17/115, A61B 19/00

(54) **Removable introducer device for delivering a medical instrument to a target in vivo site**

(71) Applicant: Myers, Stephen R., Lexington OH 44904 (US)
(72) Inventor: Myers, Stephen R., Lexington OH 44904 (US)
(74) Representative: Makovski, Priscilla Mary

(57) **Abstract**

An introducer 100 and a medical instrument 30 that is delivered to a target *in vivo* site, the combination including a medical instrument 30 having an open end 44 exposing a hollow interior, and an introducer including a tail portion 104 releasably joined to the open end of the medical instrument, a blunt tapered lead portion 106 opposite the tail portion to facilitate *in vivo* delivery of the introducer and means for receiving an *in vivo* guide wire 26.

## Description

### TECHNICAL FIELD

The present invention relates to an introducer device and the method of its use for delivering a medical instrument to an *in vivo* site. The introducer device and method are particularly useful in delivering an anvil of an anastomosis stapling device to an *in vivo* stapling site, although it will be beneficial in other medical procedures.

### BACKGROUND OF THE INVENTION

In various medical procedures, it is necessary to deliver a medical instrument to an internal or *in vivo* site. For example, anvils for surgical staplers must be delivered to *in vivo* stapling sites. Various types of surgical stapler instruments have been known for the application of staples to tissue. It has been known to use various types of staplers in gastric and esophageal surgery in both classic or modified gastric reconstructions performed end-to-end, end-to-side or side-to-side. In many cases, instruments, such as that described in U.S. Pat. No. 5,104,025, entitled "Intraluminal Anastomotic Surgical Stapler with Detached Anvil," have been used where an anvil assembly mounted on the end of a center rod can be manipulated relative to a staple assembly on the end of a tubular housing of the instrument. In particular designs of such stapling instruments, and particularly in those designs similar to that shown in the referenced '025 patent, the anvil employed has an open end exposing a hollow interior. This design makes it difficult to quickly and unintrusively deliver the anvil to the target site. This is particularly true in gastric bypass surgery and other gastrointestinal surgeries in such locations as the rectum.

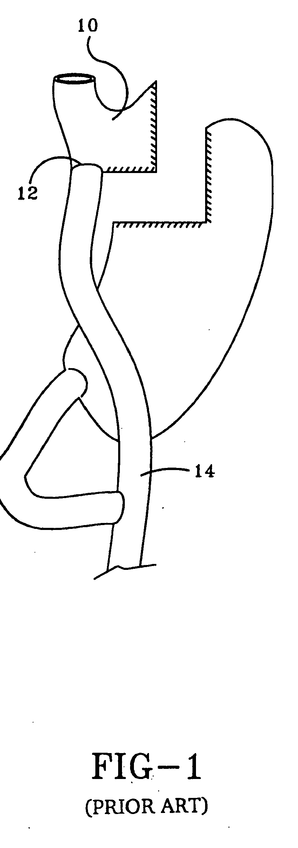

The gastric bypass operation is designed to limit the amount of food you eat. Referring to Fig. 1, the operation generally entails stapling and dividing the stomach. The "new stomach", also called the pouch (10), is only about 5-10% the size of the "old stomach" and holds less food. Food enters the pouch from the esophagus, and leaves at an opening that is formed leading from the pouch to the small intestine. This opening is called a stoma (12) and is about the size of a dime. The stoma is formed through an intestinal connection (14), called a Roux-en-Y.

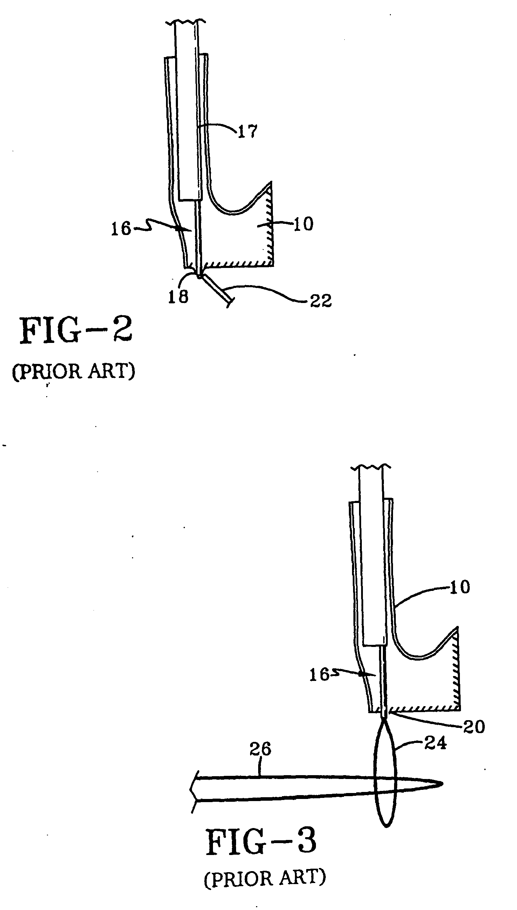

Gastric bypass is a procedure that can be performed laparoscopically, with medical instruments being delivered from points outside a patient's body to the target site where the operation is being performed. In Fig. 2, after pouch 10 is created through appropriate laparoscopic cutting and stapling techniques, a snare device 16 is introduced through an endoscope 17 that is passed through the patient's esophagus to the internal wall of pouch 10, where it is pressed to create tent 18, to be viewed by the laparoscope positioned within the abdominal cavity. Hole 20 (Fig. 3) is created in the pouch wall at tent 18 by, for example, cauterizing at tent 18 with an electric cautery device 22 (Fig. 2). Because snare device 16 is pressed against the wall of pouch 10, as the tent 18 is cauterized, the lead portion of snare device 16 extends through pouch 10, and snare loop 24 is then advanced out of snare device 16, as seen in Fig. 3.

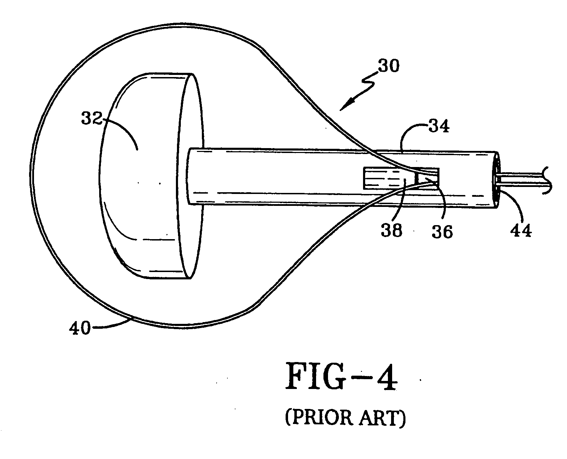

Next, a lead portion of guide wire 26, which is doubled over (folded), is threaded through snare loop 24, and upon retracting snare loop 24 into snare device 16, the folded guide wire 26 is gripped thereby and pulled outside the patient's body up through the esophagus and out through the mouth, where it is attached to a hollow stem 34 of anvil 30, shown in Fig. 4. Anvil 30 provides anvil head 32 which provides the backing surface for staples driven by an anastomosis stapler to form the gastric pouch Roux-en-Y anastomosis as known in the art. Anvil stem 34 extends from anvil head 32 and is formed with a longitudinal slot (or any similar radial aperture) 36, which in most commercial embodiments, is selectably opened at hinged cover 38, and which communicates with the hollow interior of anvil stem 34.

Guide wire 26 is fitted to anvil 30 by passing loop 40 through the open hollow end of the anvil stem and advancing loop 40 through longitudinal slot 36 and over anvil head 32, and pulling guide wire 26 until loop 40 is snug. Anvil 30 may then be delivered *in vivo* by drawing guide wire 26 back through the esophagus to hole 20 at pouch 10. Due to the design of anvil 30, with a relatively wide hollow end 44 having abrupt edges and exposing a hollow interior, drawing it down to pouch 10 can greatly irritate and even damage the patient's esophagus and or pouch 10. The edges of anvil stem 34 may catch against soft tissue, causing damage, and slowing down the anvil delivery procedure. Passing stem 34 through the wall of pouch 10, at point 18, is also difficult, time consuming, and potentially damaging. Thus, there exists a need in the art for an introducer and method for its use for delivering an anvil to a tissue site, wherein the introducer reduces the degree of resistance to introduction of the anvil, thereby reducing irritation and damage. There is also a need for such an introducer and method that may be quickly employed. More broadly, there exists a need for an introducer and method for delivering a medical instrument to a target *in vivo* site that overcomes limitations of the prior art mentioned above.

### DISCLOSURE OF THE INVENTION

The present invention provides a combination introducer and medical instrument and a method for delivering the medical instrument to an *in vivo* site. The combination includes a medical instrument having an open end exposing a hollow interior; and an introducer comprising a tail portion releasably joined to said open end of said medical instrument, a blunt tapered lead portion opposite said tail portion to facilitate *in vivo* delivery of said introducer, and means for receiving an *in vivo* guide wire.

The method is practiced with a medical instrument including an open end exposing a hollow interior, and comprising the steps of providing an introducer including a tail portion that selectively joins to the open end of the medical instrument, a blunt tapered lead portion opposite the tail portion to facilitate *in vivo* delivery of the introducer, and means for receiving an *in vivo* guide wire; advancing a drag end of an *in vivo* guide wire from a target tissue site to the introducer; securing the *in vivo* guide wire to the introducer at the drag end; securing the introducer to the medical instrument by joining the tail portion of the introducer to the open end of the medical instrument; and retracting the *in vivo* guide wire drag end toward the target tissue site to draw the introducer to the site with the medical instrument being secured thereto.

### BRIEF DESCRIPTION OF THE DRAWINGS

Figs. 1-4 depict steps of a gastric bypass operation, as known in the art;
Fig. 5 is a side view of an introducer according to this invention, shown closed, with first and second body members engaged;
Fig. 6 is a cross-sectional view taken along the line 6 - 6 of Fig. 5;
Fig. 7 is a side plan view of an introducer according to this invention, shown open, with first and second body members disengaged;
Fig. 8 is a cross-sectional view taken along the line 8 - 8 of Fig. 7;
Figs. 9 and 10 depict the joinder of the introducer and an anvil; and
Figs. 11-13 depict an alternative embodiment of an introducer and the joinder of the introducer to an anvil, with Fig. 11 showing a cross sectional view.

### DETAILED DESCRIPTION OF THE PREFERRED EMBODIMENTS

In accordance with disclosing the preferred embodiment of the present invention, the disclosure herein focuses upon an introducer and the method of its use in delivering an anvil to a *in vivo* stapling site, particularly in a gastric bypass procedure, such as that disclosed above. The invention is particularly applicable to the joining of guide wire 26 to anvil 30, and the subsequent delivery of anvil 30 to the stapling site. But this invention may have wider application in other medical procedures that will be apparent to those practitioners of ordinary skill in different medical specialties. By way of non limiting example, the introducer and method herein may find application in rectal, large/small bowel, and vascular operations.

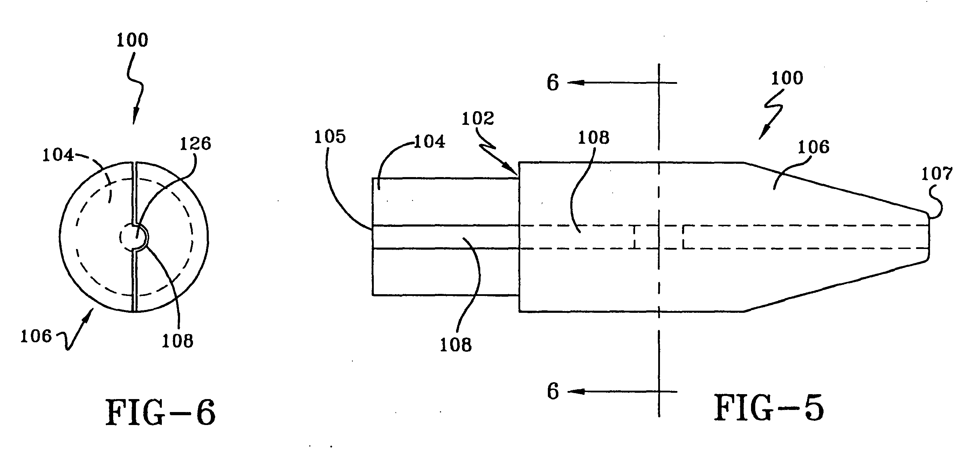

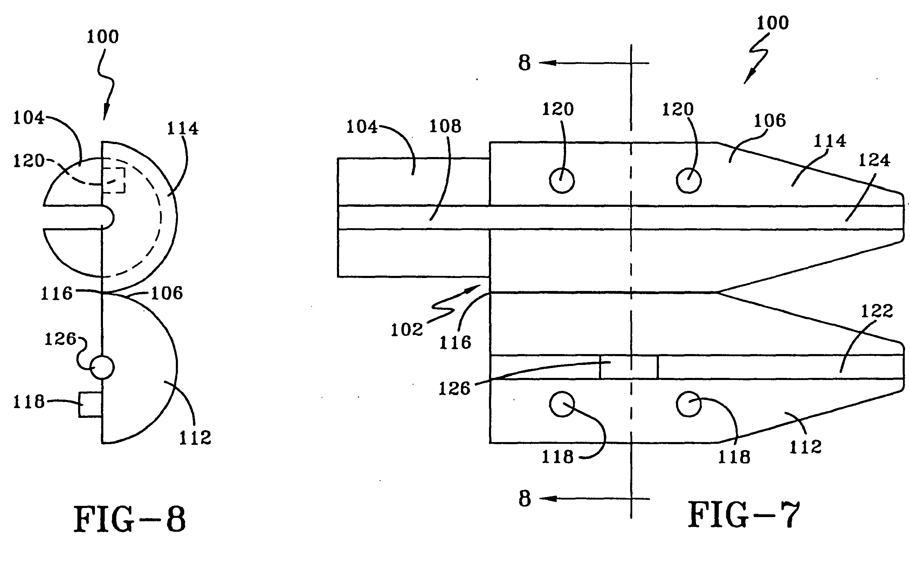

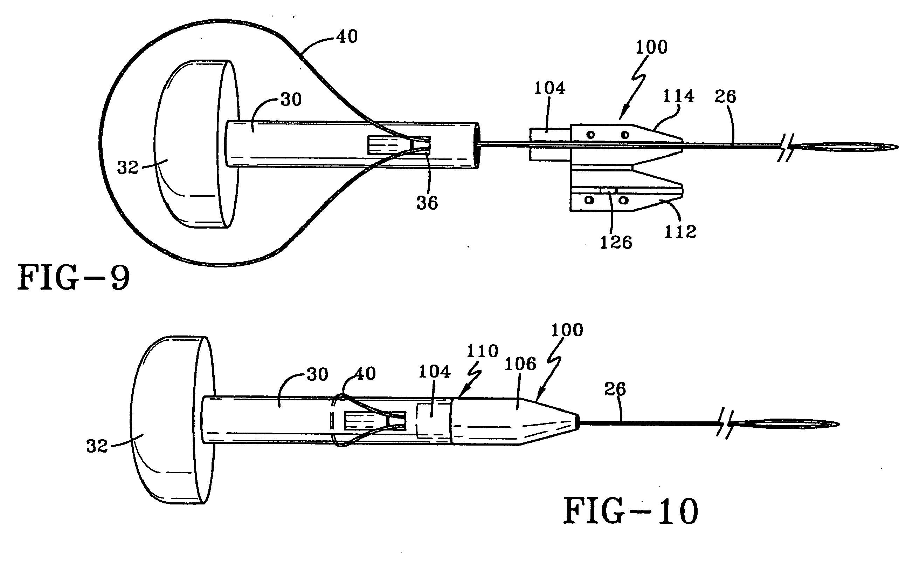

An introducer according to this invention is shown in Figs. 5-8 and designated generally by the numeral 100. Introducer 100 includes body portion 102, with tail portion 104 opposite blunt tapered lead portion 106. Axial bore 108 extends through both tail portion 104 and lead portion 106 between opposed ends 105, 107. Tail portion 104 is configured to releasably join to hollow end 44 of anvil 30 (Fig. 10), and is preferably of smaller diameter than the remainder of body portion 102 so that a smooth exterior surface is provided at the joinder 110. Blunt tapered lead portion 106 facilitates *in vivo* delivery of introducer 100 and anvil 30, as will be later described. In the preferred embodiment shown, axial bore 108 at tail portion 104 is open to the exterior surface of tail portion 104, as seen in Figs. 7 and 8.

A non tail portion of body member 102, is formed of a first body member 112 releasably joined to a second body member 114. In the preferred embodiment shown, first and second body members 112, 114, are pivotally connected as at 116, and releasably join at the mating of male snap-fit members 118 and female snap-fit members 120, which, as their names imply, join through a secure "snap" fit. First and second body members 112, 114, include axial grooves 122, 124 that, when joined, define at least a portion of axial bore 108. Tooth 126 is provided (here on first body member 112) extending from axial groove 122 into said axial bore 108 to contact the opposed inner surface of axial groove 124 and thereby clamp tightly onto a guide wire positioned within bore 108, as will be disclosed below. The joinder of male and female members 118, 120 preferably provides a smooth exterior surface (Fig. 6).

Referring now to Fig. 9, the method for using introducer 100 to deliver an anvil to an *in vivo* stapling site is described. Introducer 100 is employed once guide wire 26 is pulled up through the esophagus by the snare device, and serves to connect guide wire 26 to anvil stem 34. Loop potion 40 of guide wire 26 is advanced through longitudinal slot 36 and over anvil head 32. Guide wire 26 is pulled until loop 40 is snug against anvil stem 34. Then introducer 100, with first and second body members 112, 114 pivoted open, receives a doubled up portion of guide wire 26 in the portion of axial bore 108 provided by tail member 104 and second body member 114. Tail portion 104 is then inserted into anvil stem 34 at hollow end 44 and first body portion 112 is pivoted to snap fit with second body member 114. Tooth 126 pinches the folded portion of guide wire 26 within axial bore 108 against the interior surface of axial bore 108 to securely grip guide wire 26. Introducer 100 mates with anvil 30 to create smooth exterior surfaces, as shown in Fig. 10. Thus connected to anvil 30, introducer 100 serves as a smooth tipped guide for the lead portion of anvil stem 34, as guide wire 26 is retracted back to hole 20 at pouch 10. Due to the design of introducer 30, which smoothly joins with anvil stem 34, drawing it down to pouch 10 is less invasive to the patient, causing less injury. Because the combination introducer 100 and anvil 30 do not provide abrupt exterior surfaces, anvil stem 34 does not catch against soft tissue, and this allows the surgeon to quickly deliver anvil 30 to hole 20. The combination introducer 100 and anvil 30 pass through hole 20 more quickly and with less trauma to pouch 10. Additionally, the snap method for attaching guide wire 26 to anvil 30 is less time consuming.

As another advantage, introducer 100 is easily removed from its connection with anvil 30. Particularly, loop 40 is cut at anvil stem 34 and, because tooth 126 grips guide wire 26, pulling on guide wire 26 pulls tail portion 104 out of anvil stem 34, and introducer 100 and guide wire 26 can be removed through a port placed in the abdominal wall.

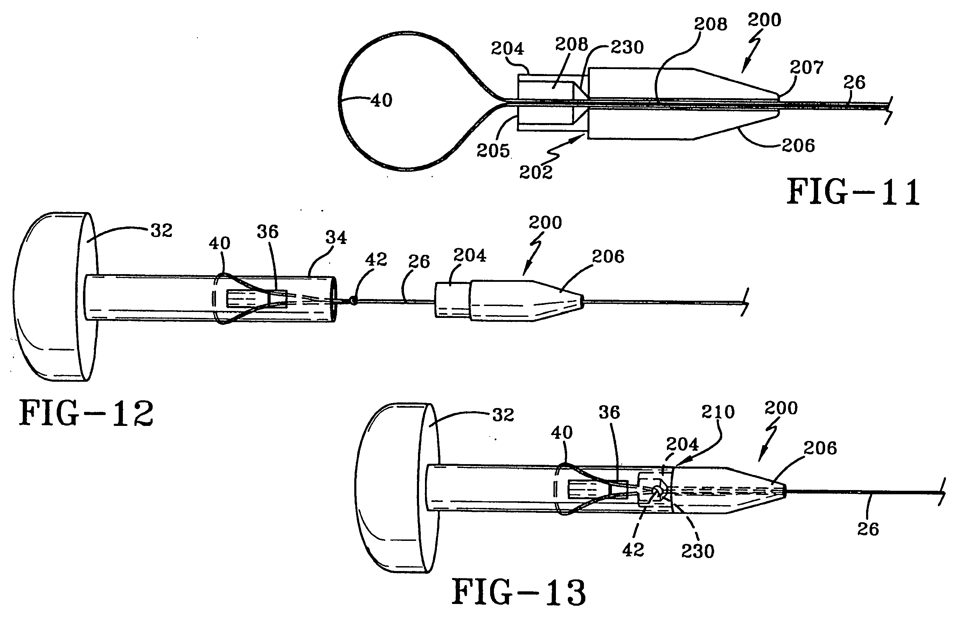

An alternative embodiment of an introducer is shown in Figs. 11-13 and designated by the numeral 200. Introducer 200 includes body portion 202 with tail portion 204 opposite blunt tapered lead portion 206. Axial bore 208 extends through both tail portion 204 and lead portion 206 between opposed ends 205, 207. Tail portion 204 is configured to be releasably joined to hollow end 44 of anvil 30 and is preferably of smaller diameter than the remainder of body portion 102, so that a smooth exterior surface is provided at the joinder 210. In this embodiment, axial bore 208 is tapered, as at 230, and body portion 202 is a single piece without multiple body members.

As before, introducer 200 is employed once guide wire 26 is pulled up through the esophagus by the snare device and serves to connect guide wire 26 to anvil stem 34. With reference to Fig. 11, guide wire 26 is folded over to create lap 40, and introducer 200 is advanced, tapered lead portion first, over the looped guide wire. Loop 40 is inserted through hollow anvil stem 34 out longitudinal slot 36 and around anvil head 32 and pulled snug. Knot 42 is then formed in guide wire 26. As in Fig. 13, introducer 200 is backed up on guide wire 26 and advanced all the way to the mating of tail portion 204 of introducer 200 with hollow end 44 of anvil 30. Thus connected, anvil 30 may be drawn back to the *in vivo* stapling site. As with the preferred embodiment of introducer 100, smooth edges are provided with the combination anvil 30 and introducer 200 and introducer 200 may be easily removed form anvil 30 by cutting at loop 40, because knot 42 is sized to wedge into the tapered portion 230 of axial bore 208.

As mentioned, the introducer and method herein may be found to be applicable in other medical procedures. Thus, more broadly, established herein is a method for delivering a medical instrument to a target *in vivo* site, wherein the medical instrument includes an open end exposing a hollow interior, the method comprising the steps of providing an introducer including a tail portion that selectively joins to the open end of the medical instrument, a blunt tapered lead portion opposite the tail portion to facilitate *in vivo* delivery of the introducer, and means for receiving an *in vivo* guide wire; advancing a drag end of an *in vivo* guide wire from a target tissue site to the introducer; securing the *in vivo* guide wire to the introducer at the drag end; securing the introducer to the medical instrument by joining the tail portion of the introducer to the open end of the medical instrument; and retracting the *in vivo* guide wire drag end toward the target tissue site to draw the introducer to the site with the medical instrument being secured thereto.

Thus, it should be evident that the introducer device and method for delivering a medical instrument to a target *in vivo* site disclosed herein carries out one or more of the objects of the present invention set forth above and otherwise constitutes an advantageous contribution to the art. As will be apparent to persons skilled in the art, modifications can be made to the preferred embodiments disclosed herein without departing from the spirit of the invention, the scope of the invention herein being limited solely by the scope of the attached claims.

## Claims

1. In combination, an introducer and a medical instrument that is delivered to a target *in vivo* site, the combination comprising:
a medical instrument having an open end exposing a hollow interior; and
an introducer comprising:
a tail portion releasably joined to said open end of said medical instrument;
a blunt tapered lead portion opposite said tail portion to facilitate *in vivo* delivery of said introducer; and
means for receiving an *in vivo* guide wire.

2. The combination of claim 1, wherein said means for receiving an *in vivo* guide wire comprises:
a first body member releasably joined to a second body member.

3. The combination of claim 2, wherein said first and second body members are pivotally connected and releasably join through a snap fit.

4. The combination of claim 2, wherein said first and second body members are joined to define an axial bore in said introducer, and said first body member provides a tooth that extends into said axial bore to contact an inner surface thereof.

5. The combination of claim 4, further comprising an *in vivo* guide wire extending into said axial bore and being secured to said introducer, at said axial bore, by said tooth.

6. The combination of claim 1, wherein said means for receiving an *in vivo* guide wire includes an axial bore within said introducer.

7. The combination of claim 6, wherein said axial bore includes a tapered section.

8. The combination of claim 7, further comprising an *in vivo* guide wire having a knot nested within said tapered section.

9. The combination of claim 1, wherein said medical instrument is an anvil for an anastomotic surgical stapler.

10. A method for delivering a medical instrument to a target tissue site, wherein the medical instrument includes an open end exposing a hollow interior, the method comprising the steps of:
providing an introducer including:
a tail portion that selectively joins to the open end of the medical instrument;
a blunt tapered lead portion opposite the tail portion to facilitate *in vivo* delivery of the introducer; and
means for receiving an *in vivo* guide wire;
advancing an *in vivo* guide wire from a target tissue site to the introducer;
securing the *in vivo* guide wire to the introducer through the means for receiving an *in vivo* guide wire;
securing the introducer to the medical instrument by joining the tail portion of the introducer to the open end of the medical instrument; and
retracting the *in vivo* guide wire toward the target tissue site to draw the introducer to the site with the medical instrument being secured thereto.

11. The method of claim 10, wherein the means for receiving an *in vivo* guide wire comprises:
a first body member releasably joined to a second body member, said first and second body members joining to define an axial bore, wherein said first body member provides a tooth that extends into said axial bore to contact an inner surface thereof, and
wherein said step of securing the *in vivo* guide wire to the introducer includes clamping the *in vivo* guide wire at the axial bore with the tooth.

12. In combination, an introducer and a medical instrument that is delivered to a target *in vivo* site, the combination comprising:
a medical instrument having an open end exposing a hollow interior having an axis; and
an introducer comprising:
a tail portion releasably joined to said open end of said medical instrument;
a blunt tapered lead portion opposite said tail portion to facilitate *in vivo* delivery of said introducer, said tail portion and said blunt tapered lead portion having axes that are coaxial with said axis of said hollow interior when said tail portion is joined to said open end of said medical instrument; and
a guide wire joined to said introducer to extend from said blunt tapered lead portion substantially along a line defining said axis of said introducer.
